(19) Europäisches Patentamt
European Patent Office
Office européen des brevets

(11) Publication number: 0 293 999 B1

# (12) EUROPEAN PATENT SPECIFICATION

(45) Date of publication of patent specification: **20.05.92**

(51) Int. Cl.5: **C07C 209/32**, C07C 231/00, C07C 211/46, C07C 233/00

(21) Application number: **88201105.9**

(22) Date of filing: **01.06.88**

The file contains technical information submitted after the application was filed and not included in this specification

## (54) Process for the coproduction of anilines and oxamides.

(30) Priority: **02.06.87 GB 8712879**

(43) Date of publication of application: **07.12.88 Bulletin 88/49**

(45) Publication of the grant of the patent: **20.05.92 Bulletin 92/21**

(84) Designated Contracting States: **BE DE FR GB IT NL**

(56) References cited:
**EP-A- 0 097 592**

**JOURNAL OF ORGANOMETALLIC CHEMISTRY, vol. 254, September 1983, pages 267-271, Elsevier Sequoia S.A., Lausanne, CH; H. HOBERG et al.: "Oxalsäurederivate durch oxidative Kupplung von Kohlenmonoxid an Nickel"**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Drent, Eit**
**Badhuisweg 3**
**NL-1031 CM Amsterdam(NL)**

EP 0 293 999 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the coproduction of anilines and oxamides, and to anilines and oxamides coproduced by this process.

The invention particularly relates to the coproduction of anilines of the formula

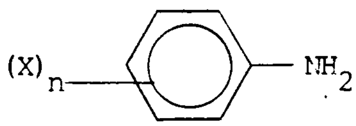

(I)

wherein X is a halogen atom, an alkyl-, alkoxy-, aryloxy-, cyano-, ester- or trifluoromethyl group and n is an integer from 0 to 5 and oxamides of the formula

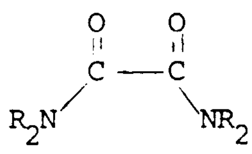

(II)

wherein each R independently is an alkyl group.

In our copending UK patent application 8615155 has been described a process for the preparation of urea derivatives by reacting an aromatic nitro compound with carbon monoxide and an amine with a specific catalyst system, comprising a group VIII noble metal, a bidentate ligand and an acid of a salt thereof.

Applicant has now found, that by varying the amount of the amine quite other and useful products have been obtained.

The invention relates to a process for the coproduction of anilines of the formula:

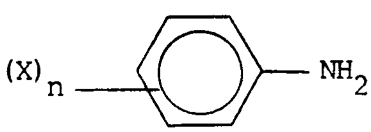

(I)

wherein X is a halogen atom, an alkyl-, alkoxy-, aryloxy-, cyano-, ester- or trifluoromethyl group and n is an integer from 0 to 5, and oxamides of the formula:

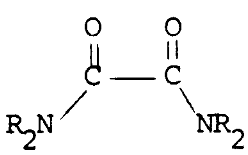

(II)

wherein each R independently is an alkyl group, which comprises reacting an aromatic nitro compound of the general formula:

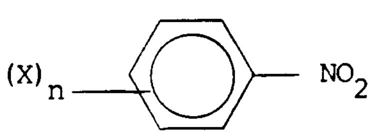

(III)

wherein X and n are as defined above with carbon monoxide and a molar excess in relation to the nitro compound of an amine $HNR_2$ wherein R is an alkyl group, in the presence of a catalyst system comprising

(a) palladium metal or a compound thereof,
(b) a bidentate ligand of the formula

$R^1R^2$--M--A--M--$R^3R^4$

in which M is P, As or Sb, A is a divalent organic bridging group having at least 2 carbon atoms in the bridge, none of these carbon atoms carrying substituents that may cause steric hindrance, and $R^1$,$R^2$,$R^3$ and $R^4$ represent similar or dissimilar optionally substituted hydrocarbon groups, and
(c) an acid or a transition metal salt of said acid.

The anilines prepared by the process according to the invention may be substituted or unsubstituted in the phenyl group in formula (I). X may be an alkyl group, preferably having from 1 to 10 carbon atoms, more preferably having from 1 to 4 carbon atoms. When X is halogen, chlorine and fluorine are preferred. Preferably the alkoxy and aryloxy groups comprise not more than 10 carbon atoms and are preferably bound to the phenyl nucleus via the oxygen atom. The ester group preferably has not more than 10 carbon atoms, more preferably not more than 6 carbon atoms. The most preferred substituents are chlorine atoms and methyl groups.

In formula (II) the groups R are preferably alkyl groups having from 1 to 10 carbon atoms, more preferably having from 1 to 4 carbon atoms, such as methyl or ethyl groups.

As already indicated the substituents X in formula I and III correspond with each other. Preferred aromatic nitro compounds are nitrobenzene, 2-chloro-4-nitro toluene and para-isopropyl nitrobenzene.

The amines used as starting materials in the process according to the invention are preferably dialkylamines with alkyl groups having from 1 to 10 carbon atoms, more preferably having from 1 to 4 carbon atoms, such as dimethylamine and diethylamine. The amines should be used in a molar excess of the amount of aromatic nitro compound, preferably the ratio of the molar amount of amine/nitro compound is 2 or more.

The reaction is presumed to occur according to the following equation

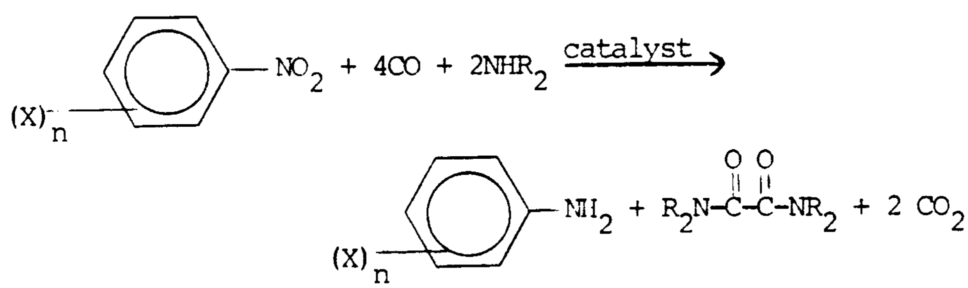


The reaction temperature may range from 50 °C to 200 °C, preferably from 70 °C to 150 °C. Pressures may range from atmospheric pressure to 200 bar.

The catalyst system comprises
(a) palladium metal or a compound thereof,
(b) a bidentate ligand of the formula

$R^1R^2$--M--A--M--$R^3R^4$

in which M is P, As or Sb, A is a divalent organic bridging group having at least 2 carbon atoms in the bridge, none of these carbon atoms carrying substituents that may cause steric hindrance, and $R^1$,$R^2$,$R^3$ and $R^4$ represent similar or dissimilar optionally substituted hydrocarbon groups, and
(c) an acid or a transition metal salt of said acid.

Both homogeneous and heterogeneous palladium compounds can be used. Homogeneous systems are preferred. Suitable palladium compounds are salts of palladium with, for example, nitric acid, sulphuric acid or alkanoic acids containing not more than 12 carbon atoms per molecule. Salts of hydrohalogenic acids can in principle be used as well. Palladium carboxylates are the preferred catalyst compounds, in particular palladium acetate. Further, palladium acetylacetonate can also be used. Palladium on carbon and palladium combined with an ion exchanger are examples of suitable heterogeneous palladium compounds.

The quantity of palladium or compound thereof is not critical. Preference is given to the use of quantities in the range of from $10^{-4}$ to $10^{-1}$ mol per mol of aromatic nitro compound.

In the bidentate ligand substituents offering steric hindrance should be absent, which means that no substituents may be present that are able to hinder the formation of complex compounds having the general formula

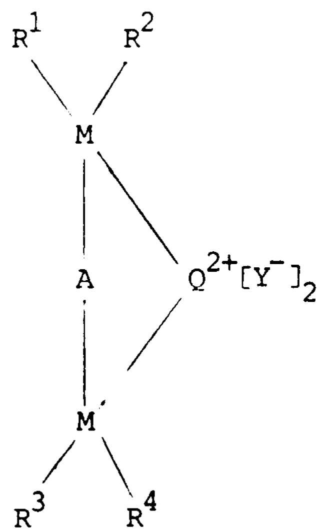

In that formula, Q represents Pd, Y represents a non-coordinating anion, whilst $Q^{2+}$ can also be written as

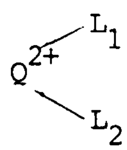
,

in which the ligands $L_1$ and $L_2$ are weakly coordinated solvent ligands, e.g. acetonitrile, methanol, acetone, or acetylacetone, or correspond with those employed in the palladium compounds described in the preceding paragraph.

In the bidentate ligand, M is preferably phosphorus. Hydrocarbon groups $R^1$, $R^2$, $R^3$ and $R^4$ will as a rule contain 2 to 18 carbon atoms, preferably 6 to 14 carbon atoms. Aryl groups are the most suitable, in particular the phenyl groups. Preferred bridging groups -A- are those having the formula $(CR^5R^6)_n$in which $R^5$ and $R^6$ are hydrogen atoms or hydrocarbon groups offering no steric hindrance and n is 3 or 4. Substituents $R^5$ and $R^6$ are preferably hydrogen atoms. The bridging groups A may also make part of cyclic structure, e.g. an aromatic or cycloaliphatic group, the carbon to carbon bond or bonds in the bridge may be saturated or unsaturated and in the bridge or in the cyclic or non-cyclic groups attached to the bridge one or more hetero atoms, e.g. sulphur, oxygen, iron or nitrogen, may have been substituted for carbon atoms, other than the two carbon atoms which must be present in the bridge linking both atoms M.

Examples of suitable bidentate ligands are
1,3-di(diphenylphosphino)propane,
1,4-di(diphenylphosphino)butane,
2,3-dimethyl-1,4-di(diphenylphosphino)butane,
1,4-di(dicyclohexylphosphino)butane,
1,3-di(di-p-tolylphosphino)propane,
1,4-di(di-p-methoxyphenylphosphino)butane,
2,3-di(diphenylphosphino)-2-butene,
1,3-di(diphenylphosphino)-2-oxopropane,
2-methyl-2-(methyldiphenylphosphino)-1,3-di(diphenylphosphino)propane,
o,o′-di(diphenylphosphino)biphenyl,
1,2-di(diphenylphosphino)benzene,
2,3-di(diphenylphosphino)naphthalene,

1,2-di(diphenylphosphino)cyclohexane,
2,2-dimethyl-4,5-di(diphenylphosphino)dioxolane and

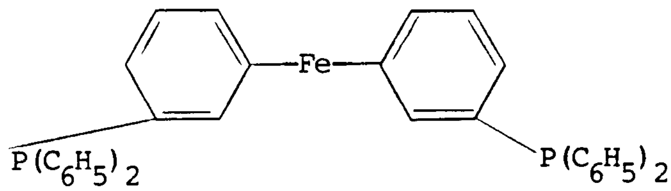

It is observed that compounds having a structure like 2-methyl-2-(methyldiphenylphosphino)-1,3-di-(diphenylphosphino)-propane, $CH_3$-C-($CH_2$-P($C_6H_5$)$_2$)$_3$, although being trifunctional, are nevertheless considered bidentate ligands in the terms of this invention since only two of the three phosphorous atoms can coordinate with the palladium atom in the complex.

The bidentate ligand can be employed in quantities which may vary within wide limits, e.g. of from 0.1 to 10 mol per mol of palladium.

The acid is preferably a carboxylic acid having 2 to 14 carbon atoms, e.g. acetic acid, 9-anthracene carboxylic acid and 2,4,6-trimethylbenzoic acid.

The transition metal which may form a salt with the carboxylic acid, may be defined as a metal of group $I^B$, $II^B$, $III^B$, $IV^B$, $V^B$, $VI^B$ and VIII, the lanthanides and actinides of the Periodic System. Preferred are copper, iron and nickel. Iron and nickel salts give about the same conversion and selectivity as those with copper salts. It is observed that with a potassium salt no conversion at all is achieved.

The process according to the invention can be carried out conveniently in the presence of a solvent. A variety of solvents can be applied such as ketones, e.g. acetone, methyl ethyl ketone, methyl isobutyl ketone, acetophenone and cyclohexanone; ethers, such as diethylene glycol dimethylether (also referred to as "diglyme"), anisole, diphenyl ether; aromatic compounds, such as benzene, toluene and the three xylenes; halogenated aromatic compounds, such as chlorobenzene and ortho-dichlorobenzene; halogenated paraffinic hydrocarbons, such as methylene chloride and carbon tetrachloride; paraffins such as hexane, heptane, cyclohexane, methylcyclohexane and iso-octane; nitriles, such as benzonitrile and acetonitrile and esters such as methyl benzoate. Aniline may be used as a solvent.

The reaction may be carried out batch-wise; semi-continuous or continuously.

The obtained oxamides are useful as fertilizers and as intermediates for fine chemicals.

## Examples

In a 250 ml Hastelloy C autoclave provided with magnetic stirrer were introduced 0.1 mol of nitrobenzene, 0.2 mol of a diethyl amine, 50 ml diethylene glycol dimethylether as solvent and the catalyst system. The autoclave was pressurized with CO at 60 bar and the temperature was raised to the required reaction temperature by electrical means. After the required reaction time the pressure was dropped, the autoclave was gradually cooled and the reaction mixture was analyzed by NMR and GLC/MS to determine the amount of aniline and oxamide.

In the following table all examples are given with the amounts of catalyst components in millimoles, the reaction times (t), the reaction temperatures (T), the conversion (calculated on nitrobenzene and the amine) and the yield of oxamide based on nitrobenzene intake (in %).

TABLE

| Example | Catalyst mmole | composition | T °C | t h | CONVERSION % AMINE | NITROBENZENE | YIELD 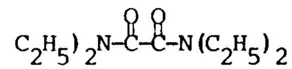 | 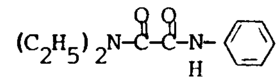 |
|---|---|---|---|---|---|---|---|---|
| 1 | 0.1<br>1<br>3 | Pd(OAc)$_2$<br>$\phi_2$-P-$(CH_2)_3$-P$\phi_2$<br>Cu (OAc)$_2$ | 80 | 1.5 | 83 | 96 | 67 | 32 |
| 2 | 0.1<br>1<br>3 | Pd(OAc)$_2$<br>$\phi_2$P-$(CH_2)_3$-P$\phi_2$<br>Cu(Ts)$_2$ | 80 | 2 | 78 | 100 | 61 | 37 |
| 3 | 0.1<br>1<br>3 | Pd(OAc)$_2$<br>$\phi_2$P-$(CH_2)_3$-P$\phi_2$<br>CuCl$_2$ | 95 | 3 | 39 | 45 | 35 | 10 |
| 4 (comparative) | 0.1<br>2<br>3 | Pd(OAc)$_2$<br>P$\phi_3$<br>Cu(OAc)$_2$ | 80 | 5 | 15 | 13 | 10 | 3 |

Ac = $CH_3$-C(O)-
$\phi$ = phenyl group
Ts = tosylate

## Claims

1. A process for the production of oxamides of the formula

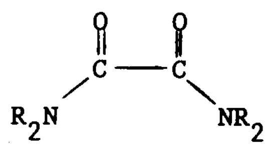

(I)

wherein each R independently is an alkyl group which comprises reacting an aromatic nitro compound of the general formula

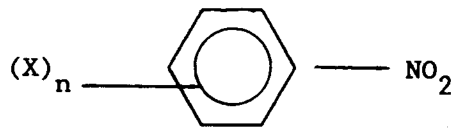

(II)

wherein X is a halogen atom, an alkyl-, alkoxy-, aryloxy-, cyano-, ester- or trifluoromethyl group and n is an integer from 0 to 5, with carbon monoxide and a molar excess in relation to the nitro compound of an amine $HNR_2$ wherein R is an alkyl group, in the presence of a catalyst system comprising
(a) palladium metal or a compound thereof,
(b) a bidentate ligand of the formula

$R^1R^2$-M-A-M-$R^3R^4$

in which M is P, As or Sb, A is a divalent organic bridging group having at least 2 carbon atoms in the bridge, none of these carbon atoms carrying substituents that may cause steric hindrance, and $R^1$ , $R^2$, $R^3$ and $R^4$ represent similar or dissimilar optionally substituted hydrocarbon groups, and
(c) an acid or a transition metal salt of said acid.

2. A process according to claim 1, wherein the ratio of the molar amount of amine to the molar amount of aromatic nitro compound is 2 or more, and anilines of the formula

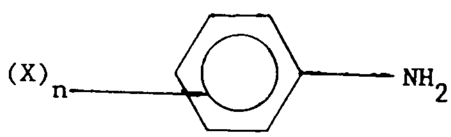

(III)

wherein X is a halogen atom, an alkyl-, alkoxy-, aryloxy-. cyano-, ester- or trifluoromethyl group and n is an integer from 0 to 5, are coproduced.

3. A process according to claim 1 or 2 wherein the amine $HNR_2$ has R groups having from 1 to 4 carbon atoms.

4. A process according to claim 3 wherein the amine is dimethylamine or diethylamine.

5. A process according to one or more of the claims 1-4 wherein the aromatic nitro compound has at least one alkyl group of 1 to 4 carbon atoms or a chlorine atom.

6. A process according to claim 5 wherein the aromatic nitro compound is p-isopropyl nitrobenzene; 3,4-dichloro nitrobenzene; 3-chloro-4-methyl nitrobenzene.

**7.** A process according to claim 1 wherein the aromatic nitro compound is nitrobenzene.

**8.** A process according to one or more of the claims 1-7 wherein a bidentate phosphine is used.

**9.** A process according to claim 8 wherein the bidentate phosphine is 1,3-di(diphenylphosphino)propane.

**10.** A process according to one or more of the claims 1 and 7-9 wherein the acid is a carboxylic acid, having 2 to 10 carbon atoms.

**11.** A process according to claim 10 wherein the carboxylic acid is acetic acid.

**12.** A process according to claim 1, 10 or 11 wherein the salt of the transition metal is a copper salt, an iron salt or a nickel salt.

**13.** A process according to any one of the preceding claims wherein the reaction takes place in the presence of a solvent.

**14.** A process according to claim 13 wherein the solvent is diethylene glycol dimethylether or aniline.

**15.** A process according to any one of the preceding claims wherein the reaction temperature is between 50 °C and 200 °C, preferably between 70 °C and 150 °C.

**Revendications**

**1.** Procédé de production d'oxamides de formule:

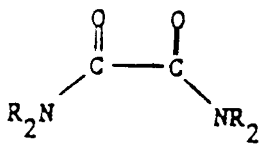

( I )

dans laquelle chaque R représente indépendamment un radical alkyle, qui consiste à faire réagir un composé nitro aromatique de formule générale :

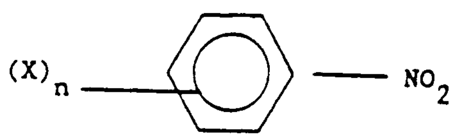

( I I )

dans laquelle X est un atome d'halogène ou un radical alkyle, alcoxy, aryloxy, cyano, ester, ou trifluorométhyle et n est un nombre entier de 0 à 5, avec de l'oxyde de carbone et un excès molaire par rapport au composé nitro d'une amine $NHR_2$ dans laquelle R est un radical alkyle, en présence d'un système catalytique comprenant :

(a) le palladium métallique ou un composé de celui-ci,

(b) un ligand bidentate de formule :

$R^1R^2$-M-A-M-$R^3R^4$

dans laquelle M représente P, As ou Sb, A représente un groupe organique divalent de pontage comportant au moins deux atomes de carbone dans le pont, aucun de ces atomes de carbone ne portant des substituants de nature à provoquer un empêchement stérique, et $R^1$, $R^2$, $R^3$ et $R^4$ sont des groupes hydrocarbonés facultativement substitués, similaires ou différents, et

(c) un acide ou un sel de métal de transition dudit acide.

2. Procédé selon la revendication 1, dans lequel le rapport de la proportion molaire de l'amine à la proportion molaire du composé nitro aromatique est de 2 ou plus et dans lequel on coproduit les anilines de formule:

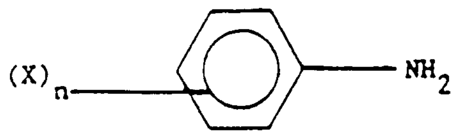

(III)

dans laquelle X est un atome d'halogène, un radical alkyle alcoxy, aryloxy, cyano, ester ou trifluorométhyle et n est un nombre entier de 0 à 5.

3. Procédé selon la revendication 1 ou 2, dans lequel l'amine $HNR_2$ comporte des groupes R contenant de 1 à 4 atomes de carbone.

4. Procédé selon la revendication 3, dans lequel l'amine est la diméthylamine ou la diéthylamine.

5. Procédé selon une ou plusieurs des revendications 1 à 4, dans lequel le composé nitro aromatique porte au moins un radical alkyle de 1 à 4 atomes de carbone ou un atome de chlore.

6. Procédé selon la revendication 5, dans lequel le composé nitroaromatique est le p-isopropylnitrobenzène, le 3,4-dichloronitrobenzène ou le 3-chloro-4-méthylnitrobenzène.

7. Procédé selon la revendication 1, dans lequel le composé nitro aromatique est le nitrobenzène.

8. Procédé selon une ou plusieurs des revendications 1 à 7, dans lequel on utilise une phosphine bidentate.

9. Procédé selon la revendication 8, dans lequel la phosphine bidentate est le 1,3-di(diphénylphosphino)-propane.

10. Procédé selon une ou plusieurs des revendications 1 et 7 à 9, dans lequel l'acide est un acide carboxylique de 2 à 10 atomes de carbone.

11. Procédé selon la revendication 10, dans lequel l'acide carboxylique est l'acide acétique.

12. Procédé selon la revendication 1, 10 ou 11, dans lequel le sel du métal de transition est un sel de cuivre, un sel de fer ou un sel de nickel.

13. Procédé selon l'une quelconque des revendications précédentes, dans lequel la réaction a lieu en présence d'un sovlant.

14. Procédé selon la revendication 13, dans lequel le solvant est l'éther diméthylique de diéthylèneglycol ou l'aniline.

15. Procédé selon l'une quelconque des revendications précédentes, dans lequel la température de réaction est comprise entre 50 et 200 ° C, de préférence entre 70 et 150 ° C.

## Patentansprüche

1. Verfahren zur Herstellung von Oxamiden der Formel

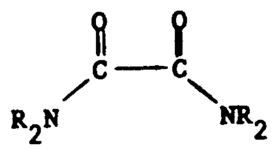

(I)

in der jedes R unabhängig voneinander eine Alkylgruppe ist, umfassend die Umsetzung einer aromatischen Nitroverbindung der allgemeinen Formel

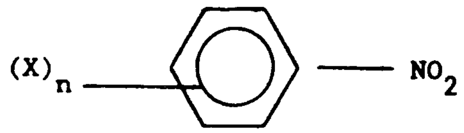

(II)

in der X ein Halogenatom, eine Alkyl-, Alkoxy-, Aryloxy-, Cyano-, Ester- oder Trifluormethylgruppe ist und n eine ganze Zahl von 0 bis 5 bedeutet, mit Kohlenmonoxid und einem molaren Überschuß in Beziehung auf die Nitroverbindung eines Amins $HNR_2$, wobei R eine Alkylgruppe ist, in Gegenwart eines Katalysatorsystems, umfassend
(a) Palladiummetall oder eine Verbindung davon,
(b) einen zweiwertigen Liganden der Formel

$R^1R^2$-M-A-M-$R^3R^4$,

in der M P, As oder Sb ist, A eine zweiwertige organische Brückengruppe mit mindestens 2 Kohlenstoffatomen in der Brücke bedeutet, wobei keines dieser Kohlenstoffatome Substituenten enthält, die zu einer sterischen Hinderung führen können, und $R^1$, $R^2$, $R^3$ und $R^4$ gleiche oder verschiedene, gegebenenfalls substituierte Kohlenwasserstoffgruppen bedeuten, und
(c) eine Säure oder ein Übergangsmetallsalz dieser Säure.

2. Verfahren nach Anspruch 1, wobei das Verhältnis der molaren Menge an Amin zu der molaren Menge an aromatischer Nitroverbindung 2 oder mehr ist, und gleichzeitig Aniline der Formel

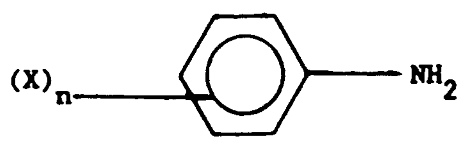

(III)

in der X ein Halogenatom, eine Alkyl-, Alkoxy-, Aryloxy-, Cyano-, Ester- oder Trifluormethylgruppe bedeutet und n eine ganze Zahl von 0 bis 5 ist, gebildet werden.

3. Verfahren nach Anspruch 1 oder 2, wobei das Amin $HNR_2$ R-Gruppen mit 1 bis 4 Kohlenstoffatomen enthält.

4. Verfahren nach Anspruch 3, wobei das Amin Dimethylamin oder Diethylamin ist.

5. Verfahren nach einem der Ansprüche 1 bis 4, wobei die aromatische Nitroverbindung mindestens eine Alkylgruppe mit 1 bis 4 Kohlenstoffatomen oder ein Chloratom enthält.

6. Verfahren nach Anspruch 5, wobei die aromatische Nitroverbindung p-Isopropyl-nitrobenzol, 3,4-Dichlor-nitrobenzol, 3-Chlor-4-methyl-nitrobenzol ist.

7. Verfahren nach Anspruch 1, wobei die aromatische Nitroverbindung Nitrobenzol ist.

8. Verfahren nach einem der Ansprüche 1 bis 7, wobei ein zweizähniges Phosphin angewandt wird.

9. Verfahren nach Anspruch 8, wobei das zweizähnige Phosphin 1,3-Di(diphenylphosphino)propan ist.

10. Verfahren nach einem der Ansprüche 1 und 7 bis 9, wobei die Säure eine Carbonsäure mit 2 bis 10 Kohlenstoffatomen ist.

11. Verfahren nach Anspruch 10, wobei die Carbonsäure Essigsäure ist.

12. Verfahren nach Anspruch 1, 10 oder 11, wobei das Salz des Übergangsmetalls ein Kupfersalz, ein Eisensalz oder ein Nickelsalz ist.

13. Verfahren nach einem der vorangehenden Ansprüche, wobei die Reaktion in Gegenwart eines Lösungsmittels abläuft.

14. Verfahren nach Anspruch 13, wobei das Lösungsmittel Diethylenglykol-dimethylether oder Anilin ist.

15. Verfahren nach einem der vorangehenden Ansprüche, wobei die Reaktionstemperatur zwischen 50 ° C und 200 ° C, vorzugsweise zwischen 70 ° C und 150 ° C, liegt.